(19) Europäisches Patentamt
European Patent Office
Office européen des brevets

(11) Publication number: **0 079 244**
**B1**

(12) **EUROPEAN PATENT SPECIFICATION**

(45) Date of publication of patent specification: **30.12.86**

(51) Int. Cl.$^4$: **C 07 D 487/04,** C 12 P 17/18, A 61 K 31/40, C 12 R 1/465 // C12R1/465 ,(C07D487/04, 209:00, 205:00)

(21) Application number: **82305975.3**

(22) Date of filing: **10.11.82**

# (54) Pluracidomycin B,C, and D and analogs thereof, their production and a microorganism for use therein.

(30) Priority: **10.11.81 JP 180628/81**
**29.12.81 JP 212435/81**
**20.01.82 JP 8144/82**
**27.08.82 JP 149544/82**

(43) Date of publication of application:
**18.05.83 Bulletin 83/20**

(45) Publication of the grant of the patent:
**30.12.86 Bulletin 86/52**

(84) Designated Contracting States:
**CH DE FR IT LI**

(56) References cited:
**EP-A-0 011 173**
**EP-A-0 024 832**
**EP-A-0 055 929**
**Chemical Abstracts vol. 97, no. 3, 19 July 1982, Columbus, Ohio, USA N. TSUJI et al. "The structures of pluracidomycins, new carbapenem antibiotics", page 403, column 2, abstract no. 20348a.**
**Patent Abstracts of Japan vol.6, no.191, 30 September 1982.**

(73) Proprietor: **SHIONOGI & CO., LTD.**
**12, Dosho-machi 3-chome Higashi-ku**
**Osaka 541 (JP)**

(72) Inventor: **Tanaka, Kentaro**
**3-19-2, Furuedai**
**Suita-shi Osaka Pref. (JP)**
Inventor: **Kondo, Eiji**
**4-22-18, Ishibashi**
**Ikeda-shi Osaka Pref. (JP)**
Inventor: **Mayama, Mikao**
**3-9-3, Hata**
**Ikeda-shi Osaka Pref. (JP)**
Inventor: **Matsumoto, Kouichi**
**6-11-7-72-401, Higashi-Toyonaka**
**Toyonaka-shi Osaka Pref. (JP)**
Inventor: **Kawamura, Yoshimi**
**1971-9, Aomadani**
**Mino-shi Osaka Pref. (JP)**
Inventor: **Tsuji, Naoki**
**14-22, Okuie-cho**
**Ashiya-shi Hyogo Pref. (JP)**

(74) Representative: **Bizley, Richard Edward et al**
**BOULT, WADE & TENNANT 27 Furnival Street**
**London EC4A 1PQ (GB)**

**EP 0 079 244 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to new antibiotics and to processes for producing the same. More particularly, it relates to new antibiotics pluracidomycin B, —C, —D, and analogs thereof and to fermentation and chemical processes for producing the same. Additionally, the invention is concerned with a new microorganism which can be used in producing the said antibiotics.

The antibiotics of this invention have the following structural formula:

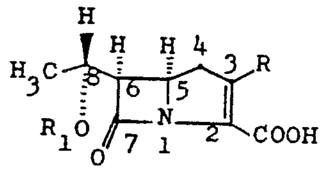

(I)

wherein either R is carboxymethylsulfinyl, formylsulfinyl, dihydroxymethylsulfinyl, hydroxymethylsulfinyl, or dihydroxymethylthio and $R_1$ is sulfo or R is sulfo and $R_1$ is hydrogen.

The compounds of the above formula are as follows: pluracidomycin B = 3-carboxymethylsulfinyl - 6 - (1 - hydroxy - sulfonyloxyethyl) - 7 - oxo - 1 - azabicyclo[3.2.0] - hept - 2 - ene - 2 - carboxylic acid (referred to as "PLM B" hereinafter); pluracidomycin C = 3 - formylsulfinyl - 6 - (1 - hydroxysulfonyloxyethyl) - 7 - oxo-azabicyclo[3.2.0] - hept - 2 - ene - 2 - carboxylic acid (referred to as "PLM C" hereinafter); dihydro pluracidomycin C = 3 - hydroxymethylsulfinyl - 6 - (1 - hydroxysulfonyloxyethyl) - 7 - oxo - 1 - azabicyclo[3.2.0] - hept - 2 - ene - 2 - carboxylic acid (referred to as "dihydro PLM C" hereinafter); deoxy pluracidomycin C = 3 - dihydroxymethylthio - 6 - (1 - hydroxysulfonyloxyethyl) - 7 - oxo - 1 - azabicyclo[3.2.0.] - hept - 2 - ene - 2 - carboxylic acid (referred to as "deoxy PLM C" hereinafter); pluracidomycin D = 3 - sulfo - 6 - (hydroxyethyl) - 7 - oxo - 1 - azabicyclo[3.2.0.] - hept - 2 - ene - 2 - carboxylic acid (referred to as "PLM D" hereinafter).

In addition, the formylsulfinyl group of PLM C turns into a dihydroxymethylsulfinyl group under a hydrated atmosphere. Both possible formulae are included in the designation "PLM C" in this specification.

This invention also includes the pharmaceutically acceptable or veterinarily acceptable salts of compounds (I) such as alkali metal salts (e.g. sodium and potassium salts), alkaline earth metal salts (e.g. calcium and barium salts) and the like.

Many carbapenem antibiotics produced by actinomycetes, such as those having a group $—CH(OSO_3H)CH_3$ at the 6 position in the place of $CH(OR_1)CH_3$ in formula (1), are known, for example, MM 4550 ($R = —SO—CH = CHNHCOCH_3$), MM 13902 ($R = —S—CH = CHNHCOCH_3$), and MM 17880 ($R = —S—CH_2CH_2NHCOCH_3$). (J. Antibiotic 34, 600—601 (1981)).

EP—A—24832 discloses carbapenem antibiotics related to those of the present invention which are synthesised by standard chemical means. In particular there is disclosed the disodium salt of (5R, 6R) - 3 - ethylthio - 6 - [(S) - 1 - hydroxysulphonyl - oxyethyl] - 7 - oxo - 1 - azabicyclo[3.2.0]hept - 2 - ene - 2-carboxylic acid and p - nitrobenzyl (5R, 6R) - 3 - (p - nitrobenzyloxycarbonyl - methylthio) - 6 - [(S) - 1 - hydroxyethyl] - 7 - oxo - 1 - aza - bicyclo[3.2.0]hept - 2 - ene - 2 - carboxylate.

PLM B, PLM C and PLM D are antibiotics which may be isolated from the fermentation broth of Streptomyces pluracidomyceticus. Deoxy PLM B (R (represents carboxymethylthio in formula I), deoxy PLM C and dihydro PLM C may be prepared by reduction of PLM B and PLM C.

The physicochemical properties of the new antibiotics are as in Table 1 and 2 below. The aforesaid Tables make reference to the various figures of the accompanying drawings.

Japanese patent applications Nos. 1981/180628 and 1982/149544, from which priority is claimed (amongst others) in this application, disclose PLM B and PLM C, and PLM D, respectively, these antibiotics being termed therein PA—41746—B and PA—41746—C, and PA—41746—D, respectively.

TABLE 1

| Item | PLM B (sodium salt) | PLM C (sodium salt) | PLM D (disodium salt |
|---|---|---|---|
| (a) Ultraviolet absorption spectrum $\lambda_{max}^{H_2O}$ nm ($E_{1\,cm}^{1\%}$) | 285 (113) (Fig. 1) | 286 (120) (Fig. 4) | 232 (108), 267 (110) |
| (b) Infrared absorption spectrum $\nu_{max}^{KBr}$ $cm^{-1}$ | 3430, 1755, 1610, 1385, 1250, 1230, 1070, 1040, 1020, 935, 900, 780, 680, 620, 585, (Fig. 2) | 3430, 1770, 1625, 1595, 1395, 1250, 1230, 1070, 1040, 1025, 937, 900, 780, 620, 585, (Fig. 5) | 3435, 1755, 1615, 1395, 1220—1200, 1105, 1066, 1059. (Fig. 7) |
| (c) Circular dichroism spectrum $\lambda_{nm}([\theta])$ | 350(0), 275 (+21500), 256(0), 312.5(−55400), 205(−4300) | 350(0), 275(+25000), 253(0), 215(−60200), 205(−38900) | ——— |
| (d) $^1$H−NMR spectrum (in $D_20$, external standard TMS) $\delta_{ppm}$ (J = Hz) *1 100 MHz *2 200 MHz | 1.97 (3H, d, J = 6), 3.68, (1H, d − d, J = 11 and 19), 3.92 (1H, d − d, J = 9 and 19), 4.36 (2H, s), 4.42 (1H, d − d, J = 6 and 9), 4.95 (1H, m), 5.38 (1H, m) (Fig. 3)*1 | 1.97 (3H, d, J = 6), 3.68 (1H, d − d, J = 11 and 19), 3.92 (1H, d − d, J = 9 and 19), 4.42 (1H, d − d, J = 6 and 9), 4.95 (1H, m), 5.38 (1H, m), 5.86 (1H, s) (Fig. 6)*1 | 0.72 (3H, d, J = 6), 2.36 (1H, d − d, J = 16.5 and 11), 2.59 (1H, d − d, J = 9 and 16.5), 3.01 (1H, d − d, J = 5.5 and 10), 3.63 (1H, q − d, J = 6.0 and 10), 3.78 (1H, m)*2 (Fig. 8) |
| (e) $^{13}$C − NMR spectrum (in $D_2O$, external standard DSS[1)]) $\delta_{ppm}$ | 177.5s, 172.3s, 166.5s, 140.6s, 140.0s, 74.0d, 60.2t, 59.1d, 54.7d, 30.5t, 19.3q. | 177.5, 166.4s, 140.5s, 140.0s, 86.5d, 73.9d, 59.2d, 54.7d, 30.4t, 19.3q | 179.4s, 169.0s, 139.8s, 127.7s, 64.4d, 60.4d, 55.0d, 32.4t, 21.7q. |
| (f) High pressure paper electrophoresis[2)] Relative Mobility [3)] | 2.7 | 1.9 | 2.3 |
| (g) Elementary analysis | C, 27.19; H, 3.38; N, 3.49; S, 12.66; Na, 13.55 | ——— | ——— |

Note: 1) δ ppm from DSS internal $CH_3CN$, δ = 1.7) DSS = sodium 2,2-dimethyl-2-silapentane-5-sulfonate
2) Toyo filter paper No. 131 (1 × 25 cm); 1/30 phosphate buffer solution (pH 7.0); 12 V/cm; 1.5 hours
3) Relative mobility is calculated from mobility of epithrenamycin A which is set as 1.

TABLE 2

| Item | | Deoxy PLM C (Na salt) | Dihydro PLM C (Na salt) |
|---|---|---|---|
| $^1H$ – NMR Spectrum (in $D_2O$, External standard TMS) $\delta_{ppm}$ (J = Hz) *1 200 MHz *2 100 MHZ | | 0.85, (3H, d, J = 6), 2.50 (1H, d – d, J = 10 and 18), 2.72 (1H, d – d, J = 9 and 18), 3.19 (1H, d – d, J = 5 and 9), 3.65 (1H, m), 4.15 (1H, m), 4.52 (1h, s)*1 | 1.97 (3H, d, J = 6.5), 3.57 (1H, d –d. J = 10.5 and 18.5), 3.90 (1H, d = d, J = 9.3 and 18.5), 4.3—4.54 (3H, m). 4.95 (1H, m), 5.36 (1H, m)*2 |
| Infrared absorption spectrum $\nu_{max}^{KBr}$ $cm^{-1}$ | | 3420, 1750, 1600, 1400, 1255, 1255, 1070, 1040, 1020, 935, 900, 780, 690, 620, 580, | 3430, 2930, 1770, 1620, 1590, 1400, 1250, 1230, 1065, 1020, 935, 895, 785, 625, 585, |

PLM B, PLM C, and PLM D can be prepared by fermentation processes. Thus, the production of these antibiotics comprises in general cultivating a PLM B−, PLM C− and/or PLM D-producing strain in a nutrient medium under aerobic conditions and isolating PLM B, PLM C, and/or PLM D from the resulting fermentation broth.

The composition of the culture medium and the conditions for fermentation follow those generally known for producing antibiotics. The medium essentially consists of carbon sources, nitrogen sources, and inorganic salts. Vitamins, precursors and other materials may optionally be added to stimulate the production of PLM B, PLM C and PLM D. Examples of such sources are glucose, starch, dextrin, glycerol, molasses, organic acids, and the like, which may be used alone or in combination. Examples of such nitrogen sources are soybean meal, corn steep liquor, meat extract, yeast extract, cotton seed flour, peptone, wheat germ, ammonium sulfate, and ammonium nitrate, and they may be used alone or in combination. Inorganic salts such as, for example, calcium carbonate, sodium chloride, potassium chloride, magnesium sulfate, cobalt chloride, various phosphate salts, or the like, may be added to the medium if the occasion demands.

Fermentation may be carried out under the same conditions as are usually employed for the production of antibiotics; for example, using a liquid medium, or submerged aerobic conditions, especially for mass production purposes. The pH of the medium is preferably about 5.5 to 8.5. The temperature may, for example, be kept at about 20—40°C, preferably about 20—30°C.

The fermentation period depends upon the scale of production. About 20 to 80 hours are required under large scale production conditions.

The antibiotics PLM B, PLM C, and PLM D can be isolated from the fermentation broth by conventional methods employed for isolating fermentation products. Any conventional method, such as, for example, filtration, centrifugation, adsorption and desorption with ion exchange resins, chromatography with various active adsorbants, and extraction with suitable organic solvents of every kind, may be used. Such procedures may, of course, be combined in an appropriate order. Suitable stabilizing agents (e.g. ethylenediamine tetraacetate disodium salt) may be added during the isolation procedure to avoid decomposition of the objective compounds.

This invention provides a new microorganism, *Streptomyces pluracidomyceticus* PA—41746, which produces PLM B, PLM C and PLM D and is an actinomycete isolated from a soil sample. The microbiological properties of the microorganism are as follows:

a) Morphological properties (cultured on Bennett's agar medium at 28°C for 14 days).

The microorganism grows well on Bennett's agar medium and forms comparatively abundant aerial hyphae on which spores are borne. Spore-bearing hyphae are formed on aerial hyphae and branch simply from main stems to side branches of which the ends are short spirals and adjacent to spore chains. The spore chains are short. The number of spores per chain is almost 20 or less. The spore surface is mostly smooth under electron microscopy, but some are observed to be rough. The spores are oval. Sporangium, flagellated spores or sclerotium are not observed. No split by fragmentation is observed in substrate hyphae.

b) Properties on various media (cultivated at 28°C for 14 days).

TABLE 3

| Medium | Growth | Aerial Hyphae | | Color of substrate Hyphae | Soluble Pigment |
|---|---|---|---|---|---|
| | | Growth | Color | | |
| Sucrose Nitrate Agar Medium | Well | None | — | Pale Yellowish brown | None |
| Glucose Asparagine Agar Medium | " | Well | Pale brown | " | " |
| Glycerol Asparagine Agar Medium | Fair | Slightly formed | White | " | " |
| Inorganic salt starch Agar Medium | Well | None | — | " | " |
| Tyrosine Agar Medium | " | None | — | " | Light brown to grayish brown |
| Nutrient Agar Medium | Slight | None | — | " | None |
| Yeast extract Malt extract Agar Medium | Well | Well | Pale brown | Yellowish brown | Yellowish brown (Slight) |
| Oatmeal Agar Medium | Fair | Fair | " | Pale Yellowish brown | None |
| Bennett's Agar Medium | Well | " | " | Yellowish brown | " |

The expressions of color depend on "Guide to color standard" published by the Japan Color Institute.

c) Physiological properties

| | |
|---|---|
| Liquefaction of gelatin | Positive |
| Hydrolysis of starch | Positive |
| Tyrosine reaction | Negative |
| Production of melanoid pigment | Negative |
| Coagulation of milk | Negetive |
| Peptonization of milk | Positive |

d) Utilization of carbohydrates

Carbohydrates producing good growth: D-xylose, D-glucose, D-fructose, sucrose, L-rhamnose.
Carbohydrate producing fair growth: L-arabinose.
Carbohydrates producing slight growth: Innositol, raffinose.

e) Growth temperature (cultured on Bennett's agar for 14 days)

10°C: slight growth without aerial hypha
28°C: good growth and good formation of aerial hyphae
37°C: good growth without aerial hypha
45°C: no growth

It is obvious from the above properties that strain PA-41746 belongs to the Genus *Streptomyces.*
A comparison of strain PA—41746 with similar strains is given below.

The references for similar strains are as follows: The actinomycetes, vol. 2 (1961), International Journal of Systematic Bacteriology vol. 18 (1968), vol. 19 (1969), vol. 22 (1972), Bergey's Manual of Determinative Bacteriology 8th edition (1974) and other references describing new species of actinomycetes. As a result, *Streptomyces daghestanicus* [Bergey's Manual of Determininative Bacteriology 8th Ed., page 814 and International Journal of Systematic Bacteriology vol. 18, page 104] was found to be the known strain most similar to strain PA—41746. A comparative test of strain PA—41746 and *Streptomyces daghestanicus* reveals the following differences set out in Table 4 below.

TABLE 4

| Item for comparison | strain PA—41746 | *St. daghestanicus* |
|---|---|---|
| Formation of Aerial Hyphae | | |
| Glucose Asparagine Agar Medium | Well | None |
| Tyrosine Agar Medium | None | Well |
| Spore Form | Oval | Short cylindrical |
| Physiological Properties | | |
| Liquefaction of Gelatin | Positive | Negative |
| Utilization of Sugar | | |
| Sucrose | Positive | Negative |
| Growth Temperature (45°C) | None | Good growth without aerial hyphae |

The above comparison clearly invites the conclusion that strain PA—41746 and the similar strain *Streptomyces daghestanicus* belong to different species.

Thus, it has been determined that strain PA—41746 is a new species belonging to the Genus *Streptomyces.* The strain PA—41746 has been named *Streptomyces pluracidomyceticus* sp. nov.

The strain PA-41746 has been deposited under the terms of the Budapest Treaty with the Fermentation Research Institute at Yatabe-machi, Tsukuba-gun, Ibaragi Pref. Japan since 6th September 1982 under accession number FERM BP—174.

This invention includes the use of the above new strain PA—41746 as well as the use of any microorganism belonging to the strain *Streptomyces pluracidomyceticus* and capable of producing antibiotics PLM B, PLM C and/or PLM D for the production of those antibiotics.

As already indicated. dihydro PLM C, deoxy PLM B and deoxy PLM C may be prepared by reduction of PLM B and PLM C. They may be prepared as follows.

Dihydro PLM C may be prepared by reduction of PLM C with a reducing agent such as lithium borohydride, sodium borohydride, potassium borohydride or the like. Sodium borohydride is most favoured. The reaction may be effected at room temperature for about 1 to 2 hours at about pH 6—8, preferably pH 7 in water or in water miscible solvent such as an alcohol (e.g. methanol or ethanol), tetrahydrofuran, dioxane or the like.

Deoxy PLM B and PLM C may also be prepared by reduction of PLM B and PLM C, respectively. Thus, deoxy PLM B and deoxy PLM C are prepared from PLM B and PLM C respectively by removal of the oxygen atom of the substituent at the 3 position. This removal may be effected by the usual methods such as, for example, refluxing with triphenyl phosphine in tetrachloremethane reduction with titanium trichloride, conversion to an alkoxysulfonium salt followed by reduction with a borohydride salt or a borocyanate salt, reduction with chromous chloride (Y. Akita et al.: Synthesis, *1977,* 792), reduction with phosphorous pentasulfide (I. W. J. Still et al.: Synthesis, *1977,* 468), or reduction with hydrogen sulfide and trifluoroacetic

anhydride (J. Drabowicz et al.: Chem. Letters, *1977,* 767). Catalytic hydrogenation is not preferred since the sulfur-containing group of the starting compound is poisonous to the catalyst and a large amount of the catalyst is required. Reduction with titanium trichloride is most preferred for a good yield and simplicity of operation. The amount of titanium trichloride used is usually from 4—9 eq. moles. The reagent is dissolved in a solvent from which dissolved air has previously been eliminated by suction under reduced pressure. The reaction is practised under a nitrogen or carbon dioxide atmosphere. Examples of the solvent are water, tetrahydrofuran, dioxane, and diglyme. The reaction duration is about 1 to 3 hours. It is preferred to keep the reaction pH at about 6—8.

The antibiotic compounds of this invention exhibit a wide range antimicrobial spectrum, being effective against both gram-positive and gram-negative bacteria.

Furthermore, they strongly inhibit β-lactamase enzymes of both the penicillinase-type and cephalosporinase-type. Thus, the compounds are useful as medicaments, veterinary drugs and disinfectants.

The results of antimicrobial tests and β-lactamase inhibitory tests are shown below in Tables 5 and 6.

(1) Anti-microbial Spectrum

TABLE 5

| Test Microorganism | Minimum Inhibitory Concentration (μg/ml) | | | | | |
|---|---|---|---|---|---|---|
| | PLM B | PLM C | PLM D | | Deoxy PLM C | Dihydro PLM C |
| *Staphylococcus aureus* 209P JC—1 | 50 | 25 | 6.25 | | 0.78 | 12.5 |
| *Streptococcus pneumoniae* I | 12.5 | 12.5 | 6.25 | | 0.2 | 3.1 |
| *Escherichia coli* NIHJ JC—2 | 6.25 | 12.5 | 6.25 | | 0.78 | 12.5 |
| *Klebsiella pneumoniae* SRL—1 | 12.5 | 25 | 12.5 | | 0.78 | 25 |
| *Klebsiella* sp. 363 (R) | 6.25 | 6.25 | 12.5 | | 0.78 | 12.5 |
| *Proteus mirabilis* PR—4 | 25 | 12.5 | 12.5 | | 0.78 | 25 |
| *Enterobacter cloacae* 233 | 100 | >100 | 12.5 | | 3.13 | >100 |
| *Pseudomonas aeruginosa* ATCC 25619 | >100 | >100 | >50 | | >100 | >100 |

Notes: Determined by agar dilution method;
Inoculum size is $10^6$ cells/ml;
Cultivation in sensitivity-disc agar at 37°C overnight.

(2) β-Lactamose inhibitory test

TABLE 6

| Source of β-lactamase | Minimum Effective *3 Concentration (μg/ml) | | | | | |
|---|---|---|---|---|---|---|
| | PLM B | PLM C | PLM D | | Deoxy PLM C | Dihydro PLM C |
| *Enterobacter cloacae* 92 *1 | 0.008 | 0.008 | 0.063 | | 0.002 | 0.008 |
| *Klebsiella* sp. 363 *2 | 0.008 | 0.004 | 8 | | 0.125 | 0.004 |

Notes: *1 producing a β-lactamase of the cephalosporinase-type
*2 producing a β-lactamase of the penicillinase-type
*3 The test compound was incubated with the enzyme at room temperature for 10 minutes prior to adding an indicator and the minimum concentration needed to inhibit a color change was determined.

The compounds of this invention may be orally or parenterally administered to humans or animals. They may be formed into tablets, capsules, powders or the like, optionally in admixture with diluents, stabilizing agents, preservatives, wetting agents, detergents or the like, for oral administration purposes. They can also be parenterally administered in the forms such as injectable formulations, ointments and suppositories. The dosage needed for these antibiotics is generally from about 1/10 to several times the dosage used for cefalotin, although this obviously depends upon the purpose of the treatment. Thus, for example, the daily dosage for a human adult is about 0.1 g to about 10 g when administered by subcutaneous injection.

The present compounds can also synergistically increase the antimicrobial activity of β-lactam antibiotics against β-lactaminase-producing bacteria because of their β-lactamase inhibitory activity. Thus, the compounds may be used with known β-lactam antibiotics such as penicillins (e.g. benzylpenicillin, phenoxymethylpenicillin, carbenicillin, ampicillin, amoxycillin and the like) and cephalosporins (e.g. cefaloridine, cefalothin, cefazorin, cefalexin, cefoxitin, cefacetrile, cefamandole, cefapirin, cefradine, cefaglycin, ceftezol, cefatrizine, cefmetazol and the like). Such mixed formulations are included in the present invention.

The invention thus includes a pharmaceutical or veterinary formulation comprising a compound of the invention formulated for pharmaceutical or veterinary use, respectively, and optionally also including β-lactam antibiotic. Such formulations may be in unit dosage form and/or may include a pharmaceutically acceptable or veterinary acceptable diluent, carrier or excipient.

The following Examples are given solely for the purposes of illustration of the present invention.

Example 1

(a) Fermentation process

A seed culture of *Streptomyces pluracidomyceticus* PA—41746 (FERM BP—174) was inoculated into a 2-litre Erlenmeyer flask containing 800 ml of a medium (0.5% soluble starch, 0.5 glucose, 0.5% polypeptone, 0.5% beef extract, 0.25% yeast extract, 0.25% sodium chloride and demineralized water (pH 7.0 before sterilization) and incubated at 28°C for 48 hours with rotation at 180 r.p.m.

800 ml portions of the above germinated broth were inoculated into 30-litre jars containing 20 litre of a medium (2.4% tomato paste, 2.4% dextrin, 1.2% dry yeast, 0.0006% cobalt chloride 6 hydrate and water (pH 7.0 before sterilization)) and incubated at 28°C for 65 hours with aeration of 20 litre/minute, internal pressure 0.2 kg/cm$^2$G and stirring of 150—350 r.p.m.

(b) Isolation process

To the fermentation broth obtained in the above process was added EDTA to produce a solution containing 50 γ/ml of EDTA. After centrifugation with a Scharples's centrifuge, the resultant supernatant (160 litre) was cooled to 10°C and mixed with a solution of benzyldimethylcetyl ammonium chloride (1.2%) in methylene chloride (40 litre) to move the active compound into the methylene chloride layer. The layer was extracted with a 3% aqueous solution of sodium iodide (3 litre) and lyophilized to give an active crude powder (60 g). The product (20 g) was subjected to gel filtration with Biogel P—2 (600 ml, Biorad Laboratories) eluted with water. The fractions showing activity against *Escherichia coli* were collected and lyophilized to give an active product (1.77 g). The product (5.3 g) was applied to gradient chromatography on QAE—Sephadex A—25 (Pharmacia Co., & Ltd.) eluted with 0—3% sodium chloride solution containing 0.05% ammonium chloride to give fraction D (160 ml), fraction C (290 ml), fraction B (130 ml) and fraction A (330 ml) successively.

(c) Purification process

Fraction A was adjusted to pH 6, condensed under reduced pressure and desalted on a column of Biogel P—2 (250 ml). The thus-obtained solution was adjusted to pH 6.5 and lyophilized to give a crude powder (230 mg) of PLM A. Fraction B was condensed under reduced pressure, desalted on a column of Biogel P—2 and the active fraction was lyophilized. The product was applied to column chromatography on Diaion HP—20AG (130 ml, Mitsubishi Kasei & Co., Ltd.) pretreated with 10% sodium chloride and eluted with 10% sodium chloride. The active fraction was condensed, desalted on a column of Biogel P—2 and lyophilized to give a powder (20 mg) of PLM B sodium salt. Fraction C was desalted on a column of Biogel P—2, adjusted to pH 6.4, condensed under reduced pressure and lyophilized to give an active substance (172 mg). The product was applied to chromatograghy on a column of HP—20AG eluted with 10% sodium chloride in the same manner as in fraction B. The fraction containing PLM C was desalted on a column of Biogel P—2. The active fractions were collected, adjusted to pH 6.4, condensed under reduced pressure and lyophilized to give a powder (15 mg) of PLM C sodium salt.

Fraction D was condensed at pH 6.8 and desalted on a column of Biogel P—2 (250 ml) and lyophilized to give a crude powder (195 mg). The product was subjected to a column chromatography on HP—20AG eluted with a 10% aqueous solution of sodium chloride. Fractions showing a single peak on high performance liquid chromatography (HPLC) were collected, adjusted to pH 6.8 and condensed under reduced pressure. The resultant residue was desalted and lyophilized to give a pure powder (15 mg) of PLM D.

Example 2

A solution of PLM C sodium salt (10 mg) in 0.05 M phosphate buffer solution (pH 7.0, 3.4 ml) was mixed with an aqueous solution (264 μl) of sodium borohydride (1.056 mg, 1.15 eq. moles) with stirring at room temperature. Sodium chloride (1 g) was added thereto after 10 minutes. The mixture was passed through a column of Diaion HP—20AG (100—200 mesh, 20 ml, Mitsubishi Kasei & Co. Ltd.) pre-treated with a 10% aqueous solution of sodium chloride. The column was eluted with the same solution of sodium chloride to give fractions; each contained 5 ml of eluate. The presence of the reduced product was checked by HPLC and active fraction Nos. 8—16 were collected and condensed to 5 ml under reduced pressure below 25°C. The precipitated sodium chloride was removed by filtration. The filtrate was applied to a column of Sephadex G—10 (30—120 μ, 300 ml) which was eluted with water. The desalted eluate was condensed to about 3 ml under reduced pressure below 25°C and lyophilized to give a colorless amorphous powder (8 mg) of 3-hydroxymethylsulfinyl-6-(1-hydroxysulfonyloxyethyl)-7-oxo-1-azabicyclo[3.2.0]-hept-2-ene-2-carboxylic acid (dihydro PLM C) as the sodium salt.

Example 3

Anhydrous titanium trichloride (17.6 mg, 5.0 eq. moles) was dissolved in distilled water (20 ml) degassed under reduced pressure. The solution was stirred under a nitrogen atmosphere and adjusted to pH 7.0 with 1N sodium hydroxide to give a black suspension. A solution of PLM B sodium salt (11 mg) in distilled water (1.5 ml) degassed under reduced pressure was added thereto. An additional suspension of anhydrous titanium trichloride (8.8 mg, 2.5 eq. moles) were prepared in the same manner as noted above was mixed therewith after 1 hour. Air was introduced into the reaction vessel after 13 minutes to oxidize the excess reagent. The resultant white precipitate was removed by centrifugation after adjustment of the reaction mixture to pH 7.0. The supernatant was condensed to 3 ml under reduced pressure below 25°C, applied to a column of Biogel P—2 (250 ml, Bio-Rad. & Co. Ltd.) and eluted with distilled water. Fractions were checked by HPLC. Those containing the reduced product were collected, condensed under reduced pressure below 25°C and lyophilized to give sodium salt of 3-carboxymethylthio-6-(1-hydroxysulfonyloxyethyl)-7-oxo-1-azabicyclo[3.2.0]-hept-2-ene-carboxylic acid (deoxy PLMB) as a pale yellowish amorphous powder (8 mg).

Example 4

A suspension of anhydrous titanium trichloride (18.6 mg, 5.0 eq. moles) in distilled water (20 ml) was prepared in the same manner as in Example 3 and mixed with a solution of PLM C sodium salt (10 mg) in distilled water (1.5 ml) degassed under reduced pressure. the mixture was stirred for 1.5 hours and air was introduced into the reaction vessel. A pale yellowish amorphous powder (6 mg) of 3-dihydroxymethylthio-6-(1-hydroxysulfonyloxyethyl)-7-1-azabicyclo[3.2.0]-hept-2-ene-carboxylic acid (deoxy PLM C) sodium salt was obtained by following the same procedure as noted in Example 3.

Example 5

The disodium salt of PLM D(0.1 g) and sodium hydrogen phosphate are dissolved in distilled water (4 ml) to give an injectable solution.

Example 6

PLM B (100 mg), corn starch (150 mg), magnesium stearate (10 mg) and talc (10 mg) are mixed, to give a powder.

**Claims**

1. A compound of the formula:

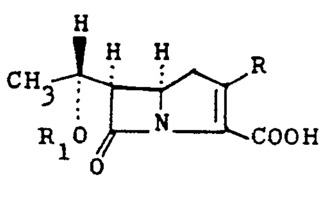

wherein either R is carboxymethylsulfinyl, formylsulfinyl, dihydroxymethylsulfinyl, hydroxymethylsulfinyl, or dihydroxymethylthio and $R_1$ is sulfo or R is sulfo and $R_1$ is hydrogen.

2. A pharmaceutically acceptable or veterinarily acceptable salt of a compound as claimed in claim 1.

3. A process for preparing a compound as claimed in claim 1 or a compound wherein in the formula of claim 1 R is carboxymethylthio which process comprises cultivating a pluracidomycin Bp, pluracidomycin C-, and/or pluracidomycin D-producing strain belonging to the species *Streptomyces pluracidomyceticus* under aerobic conditions and isolating, for example as a salt, pluracidomycin B (R is carboxymethylsulfinyl), pluracidomycin C (R is formylsulfinyl), and/or pluracidomycin D (R is sulfo and $R_1$ is hydrogen) and, optionally, converting resulting pluracidomycin B and/or C to dihydropluracidomycin C (R

is hydroxymethylsulfinyl) and/or deoxypluracidomycin B (R is carboxymethylthio) and/or deoxypluracidomycin C (R is dihydroxymethylthio) by reduction.

4. A process as claimed in claim 3, wherein the strain is *Steptomyces pluracidomyceticus* PA—41746 (FERM BP—174).

5. A process as claimed in claim 3 or claim 4 wherein the cultivation is effected at 20—40°C.

6. A process as claimed in any one of claims 3 to 5, wherein pluracidomycin C is isolated and reduced with lithium borohydride, sodium borohydride or potassium borohydride to give dihydropluracidomycin C.

7. A process as claimed in any one of claims 3 to 5, wherein pluracidomycin B is isolated and reduced with titanium trichloride to give deoxypluracidomycin B.

8. A process as claimed in any one of claims 3 to 5, wherein pluracidomycin C is isolated and reduced with titanium trichloride to give deoxypluracidomycin C.

9. *Streptomyces pluracidomyceticus* PA—41746 (FERM BP—174).

10. A pharmaceutical or veterinary formulation comprising a compound as claimed in claim 1 or a salt as claimed in claim 2 formulated for pharmaceutical or veterinary use, respectively, and optionally also including a β-lactam antibiotic.

11. A compound as claimed in claim 1, a salt as claimed in claim 2, or a formulation as claimed in claim 10, in each case for use against gram-positive and/or gram-negative bacteria as a drug.

**Patentansprüche**

1. Verbindung der Formel

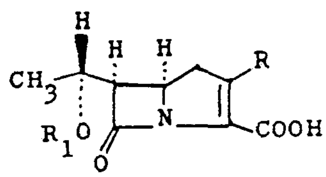

worin entweder R Carboxymethylsulfinyl, Formylsulfinyl, Dihydroxymethylsulfinyl, Hydroxymethylsulfinyl oder Dihydroxymethylthio und $R_1$ Sulfo bedeuten oder R Sulfo und $R_1$ Wasserstoff bedeutet.

2. Pharmazeutisch annehmbares oder veterinär annehmbares Salz einer Verbindung gemäss Anspruch 1.

3. Verfahren zur Herstellung einer Verbindung gemäss Anspruch 1 oder einer Verbindung, in welcher in der Formel von Anspruch 1 R Carboxymethylthio bedeutet, wobei das Verfahren umfasst: Kultivieren eines Pluracidomycin B-, Pluracidomycin C- und/oder Pluracidomycin D-produzierenden Stammes, der zu der Spezies Streptomyces pluracidomyceticus gehört, unter aeroben Bedingungen und Isolieren, beispielsweise als ein Salz, von Pluracidomycin B (R ist Carboxymethylsulfinyl), Pluracidomycin C (R ist Formylsulfinyl) und/oder Pluracidomycin D (R ist Sulfo und $R_1$ ist Wasserstoff) und gewünschtenfalls das Umwandeln des gebildeten Pluracidomycins B und/oder C in Dihydropluracidomycin C (R ist Hydroxymethylsulfinyl) und/oder Desoxypluracidomycin B (R ist Carboxymethylthio) und/oder Desoxypluracidomycin C (R ist Dihydroxymethylthio) durch Reduktion.

4. Verfahren gemäss Anspruch 3, worin der Stamm Streptomyces pluracidomyceticus PA—41746 (FERM BP—174) ist.

5. Verfahren gemäss Anspruch 3 oder Anspruch 4, bei dem die Kultivierung bei 20 bis 40°C durchgeführt wird.

6. Verfahren gemäss einem der Ansprüche 3 bis 5, bei dem Pluracidomycin C isoliert und mit Lithiumborhydrid, Natriumborhydrid oder Kaliumborhydrid unter Erhalt von Dihydropluracidomycin C reduziert wird.

7. Verfahren gemäss einem der Ansprüche 3 bis 5, bei dem Pluracidomycin B isoliert und mit Titantrichlorid unter Erhalt von Desoxypluracidomycin B reduziert wird.

8. Verfahren gemäss einem der Ansprüche 3 bis 5, bei dem Pluracidomycin C isoliert und mit Titantetrachlorid under Erhalt von Desoxypluracidomycin C reduziert wird.

9. Streptomyces pluracidomyeticus PA—41746 (FERM BP—174).

10. Pharmazeutische oder veterinäre Formulierung, umfassend eine Verbindung gemäss Anspruch 1 oder ein Salz gemäss Anspruch 2, die für eine pharmazeutische bzw. eine veterinäre Anwendung formuliert ist und die gewünschtenfalls auch ein β-Lactam-Antibiotikum einschliesst.

11. Verbindung gemäss Anspruch 1, ein Salz gemäss Anspruch 2 oder eine Formulierung gemäss Anspruch 10, die jeweils als ein Arzneimittel für die Verwendung gegen gram-positive und/oder gram-negative Bakterien vorliegt.

**Revendications**

1. Composé de formule:

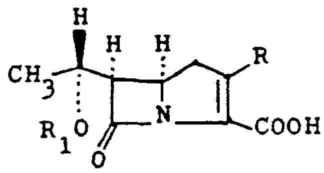

dans lequel soit R est carboxyméthylsulfinyle, formylsulfinyle, dihydroxyméythylsulfinyle, hydroxyméthylsulfinyle, ou dihydroxyméthylthio et $R_1$ est sulfo soit R est sulfo et $R_1$ est hydrogène.

2. Un sel pharmaceutiquement acceptable ou vétérinairement acceptable d'un composé selon la revendication 1.

3. Procédé de préparation d'un composé selon la revendication 1 ou d'un composé dans lequel, dans la formule de la revendication 1, R est carboxyméthylthio, procédé qui comprend la culture d'une souche produisant de la pluracidomycine B, de la pluracidomycine C, et/ou de la pluracidomycine D, souche appartenant à l'espèce *Streptomyces pluracidomyceticus,* dans des conditions d'aérobie et l'isolement, par exemple en un sel, de pluracidomycine B (R est carboxyméthylsulfinyle), de pluracidomycine C (R est formylsulfinyle), et/ou de pluracidomycine D (R est sulfo et $R_1$ est hydrogène) et, facultativement, la conversion par réduction de la pluracidomycine B et/ou C résultantes en dihydropluracidomycine C (R est hyroxyméthylsulfinyle) et/ou en déoxypluracidomycine B (R est carboxyméthylthio) et/ou en déoxypluracidomycine C (R est dihydroxyméthylthio).

4. Procédé selon la revendication 3, dans lequel la souche est *Streptomyces pluracidomyceticus* PA—41746 (FERM BP—174).

5. Procédé selon la revendication 3 ou la revendication 4 dans lequel la culture est effectuée à 20—40°C.

6. Procédé selon l'une quelconque des revendications 3 à 5, dans lequel la pluracidomycine C est isolée et réduite avec du borohydrure de lithium, du borohydrure de sodium ou du borohydrure de potassium pour donner la dihydropluracidomycine C.

7. Procédé selon l'une quelconque des revendications 3 à 5 dans lequel la pluracidomycine B est isolée et réduite avec du trichlorure de titane pour donner la déoxypluracidomycine B.

8. Procédé selon l'une quelconque des revendications 3 à 5 dans lequel la pluracidomycine C est isolée et réduite avec du trichlorure de titane pour donner la déoxypluracidomycine C.

9. *Streptromyces pluracidomyceticus* PA—41746 (FERM BP—174).

10. Formulation pharmaceutique ou vétérinaire comprenant un composé selon la revendication 1 ou un sel selon la revendication 2, formulée pour une utilisation pharmaceutique ou vétérinaire, respectivement, et facultativement comprenant aussi un antibiotique β-lactame.

11. Composé selon la revendication 1, sel selon la revendication 2, ou formulation selon la revendication 10, dans chaque cas pour une utilisation en tant que médicament contre les bactéries gram-positives et/ou gram-négatives.

FIG. 1.

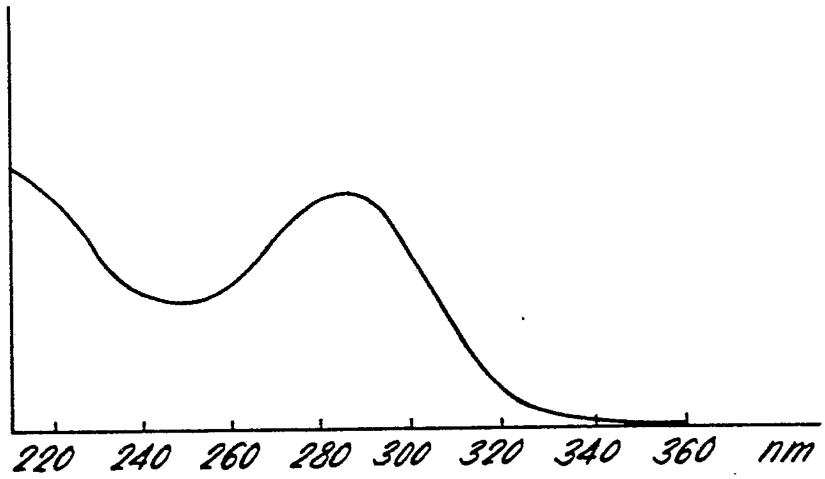
220
240
260
280
300
320
340
360
nm

FIG. 2.

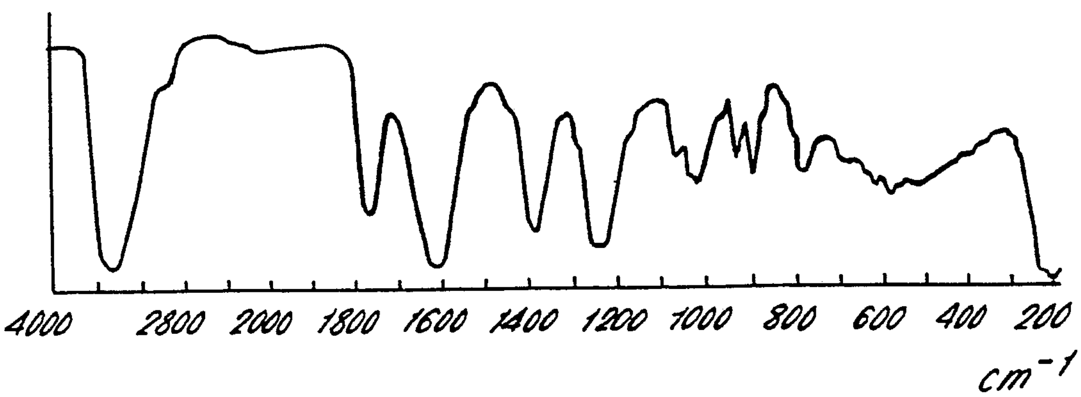
4000
2800
2000
1800
1600
1400
1200
1000
800
600
400
200
cm-1

FIG. 3

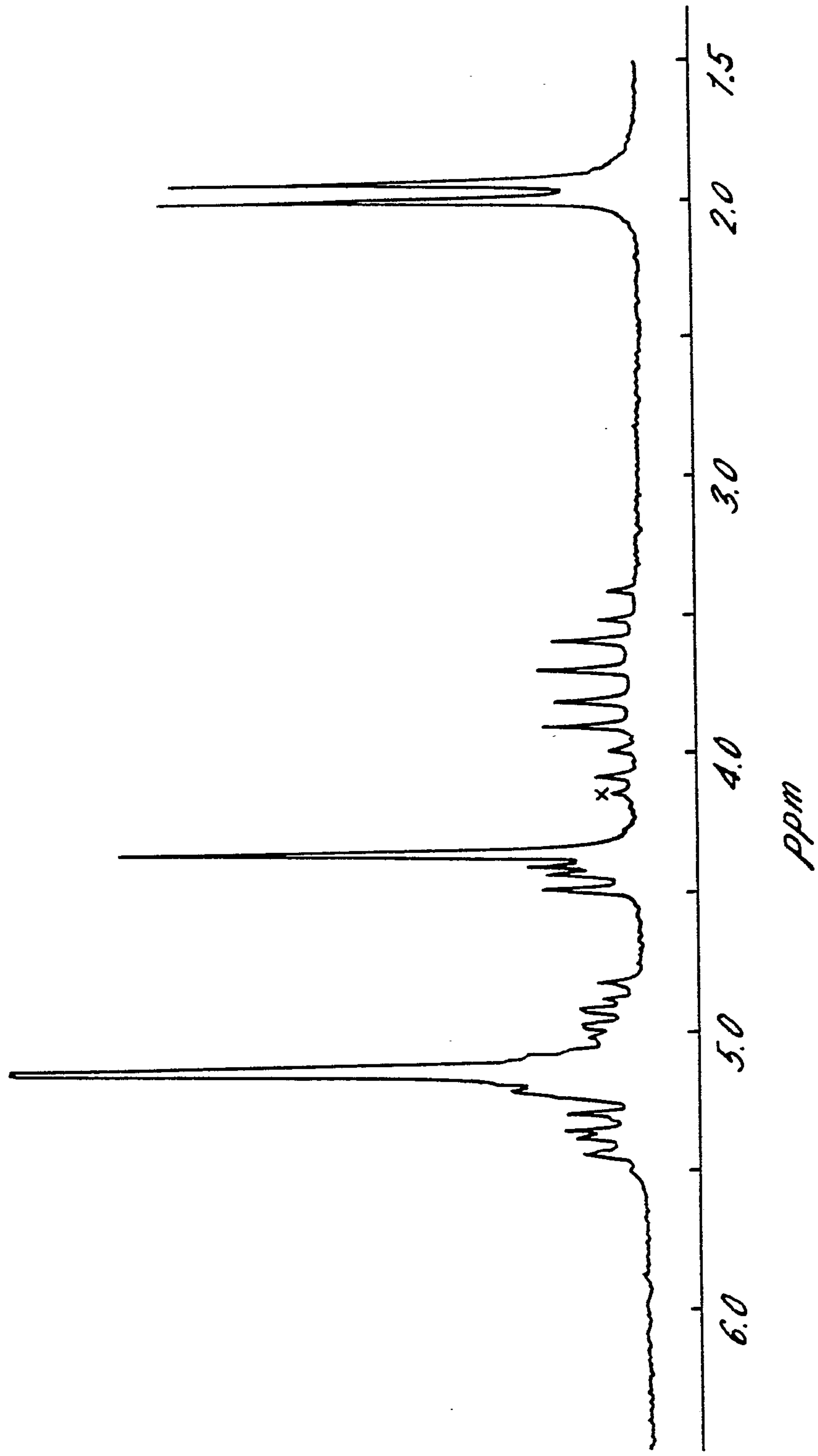

FIG. 4.

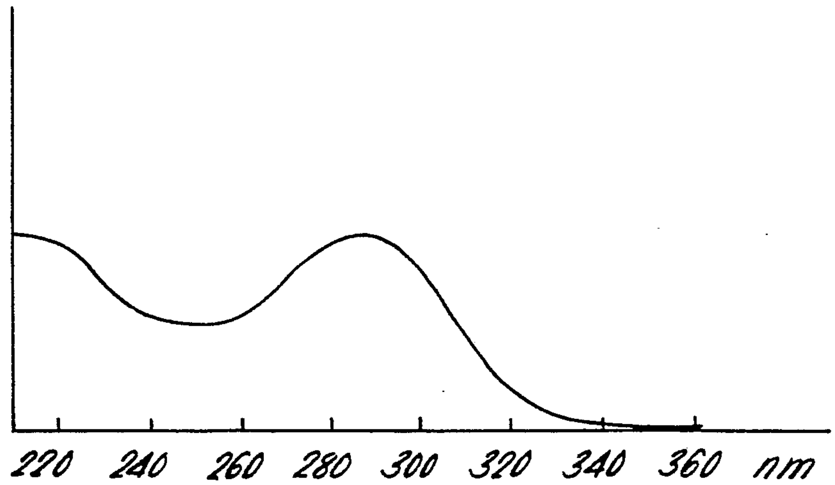
220 240 260 280 300 320 340 360 nm

FIG. 5.

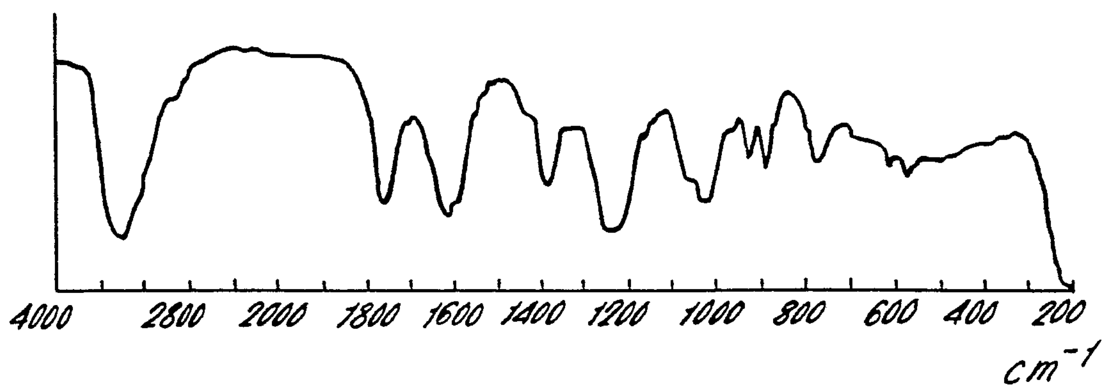
4000 2800 2000 1800 1600 1400 1200 1000 800 600 400 200
cm$^{-1}$

FIG. 6.

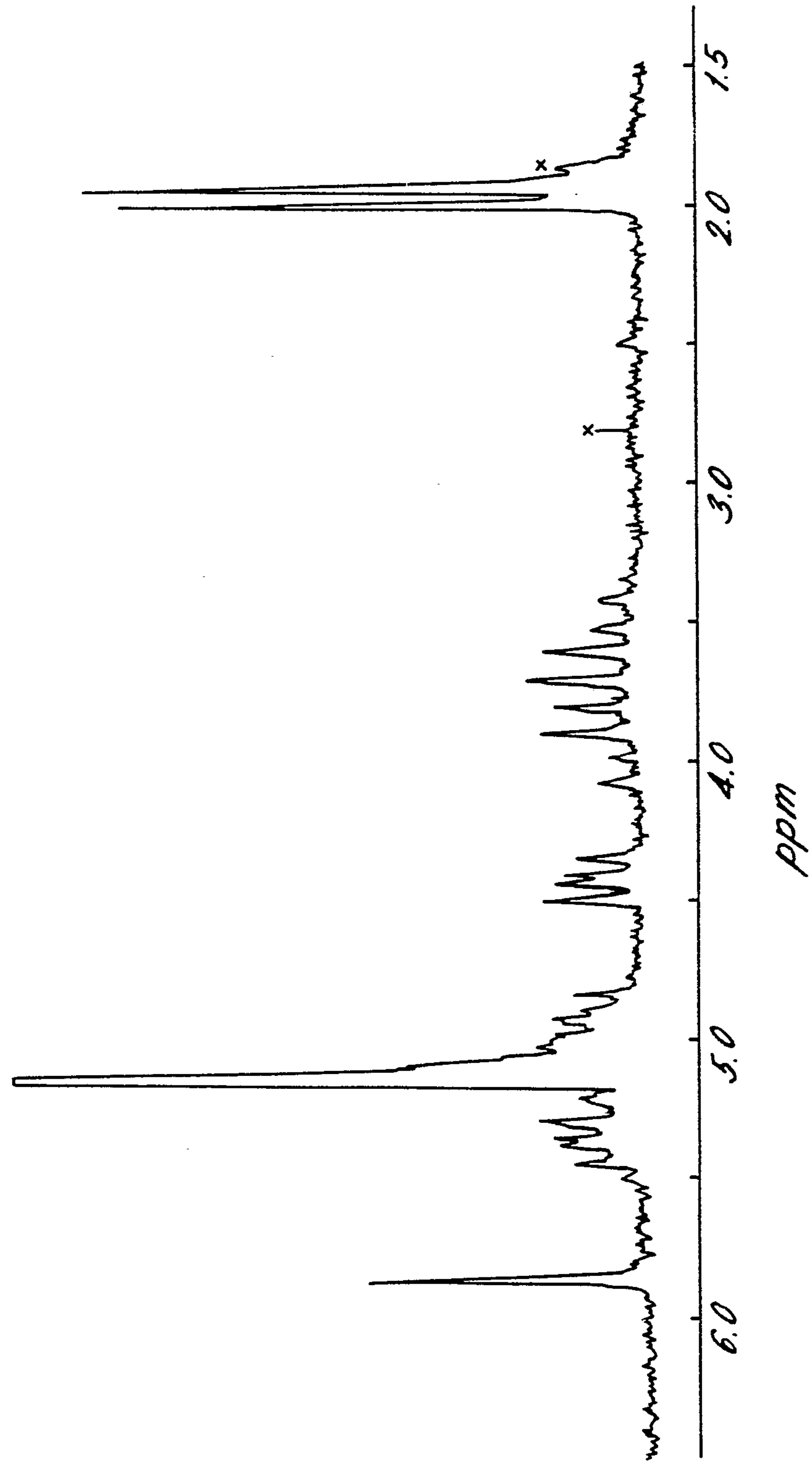

FIG. 7.

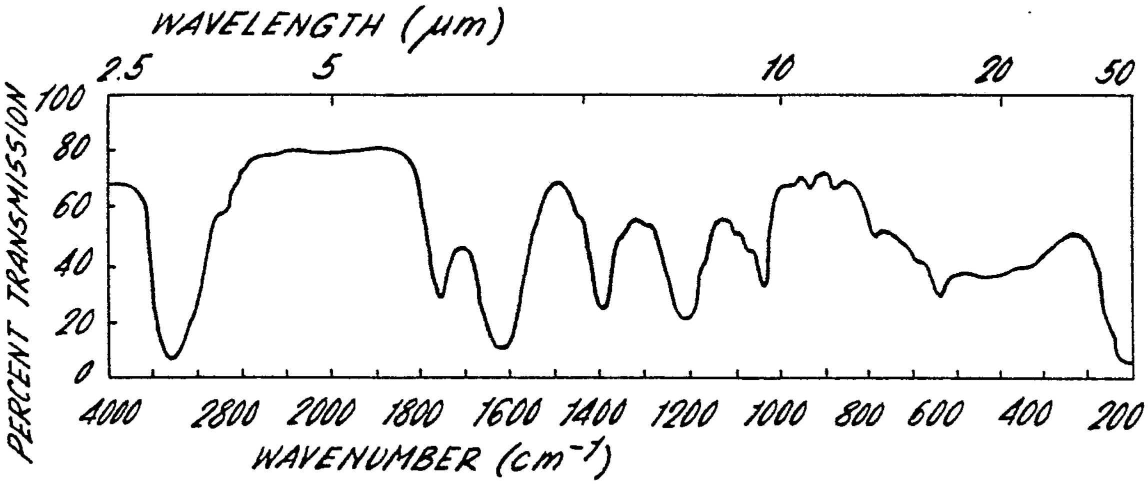
WAVELENGTH (μm)
2.5
5
10
20
50
PERCENT TRANSMISSION
100
80
60
40
20
0
4000
2800
2000
1800
1600
1400
1200
1000
800
600
400
200
WAVENUMBER (cm⁻¹)

FIG. 8.

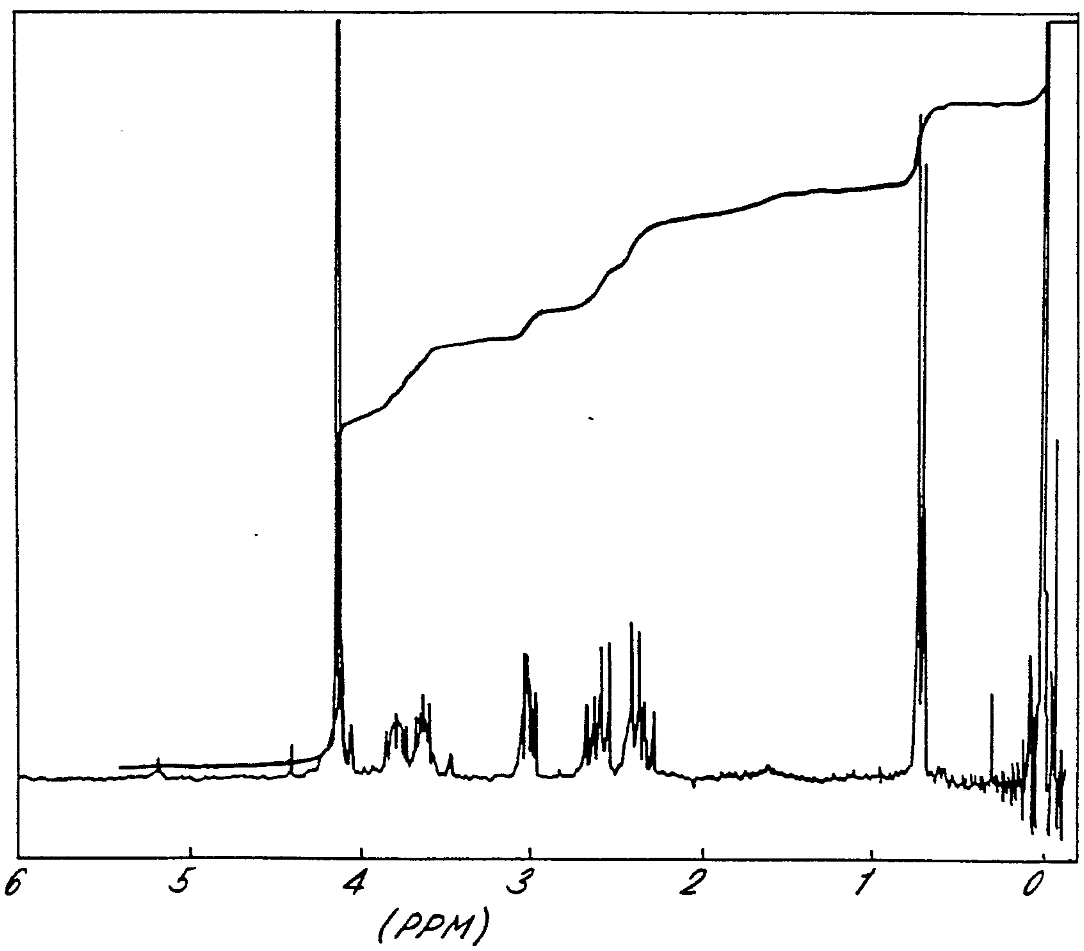
6
5
4
3
2
1
0
(PPM)